# EUROPÄISCHE PATENTSCHRIFT

(11) **EP 2 869 920 B1**
(45) Veröffentlichungstag und Bekanntmachung des Hinweises auf die Patenterteilung: **06.04.2016**
(21) Anmeldenummer: 13733297.9
(22) Anmeldetag: 02.07.2013
(51) Int. Cl.: B01J 29/76, B01J 37/02, B01J 35/04, B01J 35/02, C07C 11/167, C10G 70/02

(54) **KATALYSATOR UND VERFAHREN ZUR ENTFERNUNG VON SAUERSTOFF AUS KOHLENWASSERSTOFFSTRÖMEN**
CATALYST AND METHOD FOR REMOVING OXYGEN FROM HYDROCARBON FLOWS
CATALYSEUR ET PROCÉDÉ DE DÉSOXYGÉNATION DE FLUX D'HYDROCARBURES

(30) Priorität: 03.07.2012 EP 12174717
(43) Veröffentlichungstag der Anmeldung: 13.05.2015
(73) Patentinhaber: BASF SE, 67056 Ludwigshafen am Rhein (DE)
(72) Erfinder: REZAI, Alireza, 68163 Mannheim (DE); AVERLANT, Gauthier Luc Maurice, 60323 Frankfurt (DE); KUBANEK, Petr, 68163 Mannheim (DE); DIETERLE, Martin, 67059 Ludwigshafen (DE); HEIDEMANN, Thomas, 68519 Viernheim (DE)
(74) Vertreter: BASF IP Association
(86) Internationale Anmeldenummer: PCT/EP2013/063887
(87) Internationale Veröffentlichungsnummer: WO 2014/006017

(56) Entgegenhaltungen:
- WO-A1-2004/033598
- US-A1- 2007 004 926
- YANG H ET AL: "Incorporating platinum precursors into a NaA-zeolite synthesis mixture promoting the formation of nanosized zeolite", MICROPOROUS AND MESOPOROUS MATERIALS, ELSEVIER SCIENCE PUBLISHING, NEW YORK, US, Bd. 117, Nr. 1-2, 1. Januar 2009 (2009-01-01), Seiten 33-40, XP025658742, ISSN: 1387-1811, DOI: 10.1016/J.MICROMESO.2008.06.009 [gefunden am 2008-06-14]

## Beschreibung

Die Erfindung betrifft einen Katalysator und Verfahren zur Entfernung von Sauerstoff aus freien Sauerstoff enthaltenden Kohlenwasserstoffströmen, sowie die entsprechende Verwendung des Katalysators.

In unterschiedlichen chemischen Verfahren können freien Sauerstoff enthaltende Kohlenwasserstoffströme anfallen, aus denen der freie Sauerstoff entfernt werden soll oder muss.

Beispielsweise kann in einem ethylenisch ungesättigte Kohlenwasserstoffe enthaltenden Gasstrom enthaltender freier Sauerstoff zur Bildung von Peroxiden führen, die unter Sicherheitsaspekten schwierig zu handhaben sind.

Die WO 2006/075025 beschreibt ein Verfahren zur Herstellung von Butadien aus n-Butan durch nicht-oxidative, katalytische Dehydrierung von n-Butan, nachfolgende oxidative Dehydrierung und Aufarbeitung des Produktgemisches. Nach der oxidativen Dehydrierung kann der im Produktgasstrom verbliebene Sauerstoff entfernt werden, beispielsweise indem er katalytisch mit Wasserstoff umgesetzt wird. Ein entsprechender C₄-Produktgasstrom kann dabei 20 bis 80 Vol.-% Butadien, 20 bis 80 Vol.-% n-Butan, 5 bis 50 Vol.-% 2-Buten und 0 bis 20 Vol.-% 1-Buten sowie geringe Mengen Sauerstoff enthalten. Der Rest-Sauerstoff kann sich insoweit als störend auswirken, als er in nachgelagerten Verfahrensschritten als Initiator für Polymerisationsreaktionen wirken kann. Diese Gefahr ist insbesondere bei der destillativen Abtrennung von Butadien gegeben und kann dort zu Ablagerungen von Polymeren (Bildung von so genanntem "Popcorn") in der Extraktiv-Destillationskolonne führen. Deshalb wird eine SauerstoffEntfernung unmittelbar nach der oxidativen Dehydrierung durchgeführt, im Allgemeinen durch eine katalytische Verbrennungsstufe, in der Sauerstoff mit dem im Gasstrom enthaltenen Wasserstoff in Gegenwart eines Katalysators umgesetzt wird. Hierdurch wird eine Verringerung des Sauerstoffgehalts bis auf geringe Spuren erreicht. Als geeigneter Katalysator wird α-Aluminiumoxid beschrieben, das 0,01 bis 0,1 Gew.-% Platin und 0,01 bis 0,1 Gew.-% Zinn enthält. Alternativ werden auch Kupfer in reduzierter Form enthaltende Katalysatoren angegeben.

Die WO 2010/130610 beschreibt ein Verfahren zur Herstellung von Propylenoxid durch Umsetzung von Propen mit Wasserstoffperoxid, Abtrennung des Propylenoxids unter Erhalt eines Propen und Sauerstoff enthaltenden Gasgemisches. Diesem Gasgemisch wird Wasserstoff zugesetzt, und der enthaltene Sauerstoff wird zumindest teilweise durch Umsetzung mit dem Wasserstoff in Gegenwart eines kupferhaltigen Katalysators umgesetzt. Dabei enthält der Katalysator 30 bis 80 Gew.-% Kupfer, berechnet als CuO.

WO 2004/033598 A1 beschreibt ein Verfahren zur Entfernung von Sauerstoff aus Olefin enthaltenen Prozessströmen ohne signifikante Hydrierung der Olefine. Das Verfahren beinhaltet das Kontaktieren der Gasmischung in einer Reaktionszone mit einem Katalysator, wobei der Katalysator zumindest ein Metall oder Metalloxid umfasst aus der Gruppe bestehend aus den Elementen der 10. und 11. Gruppe des Periodensystems.

US 2007/0004926 A1 beschreibt ein Verfahren zur Herstellung von Propylenoxid umfassend die Schritte (I) bis (III). In dem Schritt (I) wird Propen mit Wasserstoffperoxid in Gegenwart eines Katalysators zur Reaktion gebracht, um eine Mischung (GI) zu erhalten. Diese Mischung enthält Propylenoxid, unreagiertes Propen und Sauerstoff. In dem Schritt (II) wird das Propylenoxid von dieser Mischung getrennt, um eine Mischung zu erhalten, die Propen und Sauerstoff enthält. In Schritt (III) wird der Gehalt an Sauerstoff reduziert, der in der Mischung von Propen und Sauerstoff aus dem vorherigen Schritt enthalten ist, indem eine Reaktion mit Wasserstoff in Gegenwart eines Katalysators, umfassend Zinn und mindestens ein Edelmetall, stattfindet.

Yang et al. ("Incorporating platinum precursors into a NaA-zeolite synthesis mixture promoting the formation of nanosized zeolite", Microporous and Mesoporous Materials 117 (2009), Seiten 33 bis 40) beschreibt ein NatriumA-Zeolith mit verschiedenen Platingehalten, wobei das Platin während der Zeolith-Synthese zugegeben wird.

EP 0 764 466 A2 beschreibt ein Katalysatormaterial, um CO zu entfernen, enthaltend einen Typ-A-Zeolithen, auf dem mindestens ein Metall, ausgewählt aus der Gruppe Pt, Pd, Ru, Au, Rh und Ir oder Legierungen von zwei oder mehr dieser Metalle, aufgebracht ist.

US 4,191,663 A beschreibt ein Herstellungsverfahren für einen synthetischen kristallinen Alumino-Silikat-Zeolithen.

Du et al. "Porosity of microporous zeolites A, X and ZSM-5 studied by small angle X-ray scattering and nitrogen adsorption", JOURNAL OF PHYSICS AND CHEMISTRY OF SOLIDS, PERGAMON PRESS, LONDON, GB, Bd. 68, Nr. 9, 21. August 2007 (2007-08-21), Seiten 1692-1699, ISSN: 0022-3697, untersuchen die Porosität von mikroporösen Zeolithen A, X und ZSM-5.

Neben der "Popcorn"-Bildung können in kohlenwasserstoffhaltigen Gasgemischen, insbesondere Butadien und Sauerstoff enthaltenden Gasgemischen, der Sauerstoffgehalt zur Desaktivierung von Katalysatoren, zu Rußablagerungen, Peroxid-Bildungen, zur Verschlechterung der Adsorptionseigenschaften von Lösungsmitteln im Aufarbeitungsprozess beitragen.

Insbesondere bei der Herstellung von Butadien aus n-Butan bildet die selektive Sauerstoffentfernung eine Grundvoraussetzung für die wirtschaftliche Durchführbarkeit des Verfahrens, da jeder Verlust des Zielproduktes Butadien mit höheren Kosten verbunden ist. Die zu erfüllende Spezifikation liegt bei einer Rest-Sauerstoff-Konzentration nach der Sauerstoffentfernungsstufe von weniger als 100 ppm.

Aufgabe der vorliegenden Erfindung ist die Bereitstellung verbesserter Katalysatoren für die katalytische Sauerstoff-Abtrennung aus Kohlenwasserstoff-Gemischen. Der Katalysator soll es erlauben, bei einem Gehalt an freiem Wasserstoff im Kohlenwasserstoffstrom die selektive Umsetzung von freiem Sauerstoff mit freiem Wasserstoff zu katalysieren, ohne dass dabei nennenswerte Mengen an Kohlenwasserstoffen, insbesondere Butadien, mit umgesetzt werden. Auch in Abwesenheit von freiem Wasserstoff soll der Gehalt an freiem Sauerstoff vermindert werden.

Die Aufgabe wird erfindungsgemäß gelöst durch einen Katalysator, enthaltend auf Zeolith A als Träger 0,01 bis 0,5 Gew.-% Platin, bezogen auf den Katalysator, dadurch gekennzeichnet, dass die BET-Oberfläche des Katalysators 20 bis 40 m²/g beträgt.

Die Aufgabe wird zudem erfindungsgemäß gelöst durch Verwendung des Katalysators zur Entfernung von Sauerstoff aus einem freien Sauerstoff enthaltenden Kohlenwasserstoffstrom durch katalytische Verbrennung in Anwesenheit oder Abwesenheit von freiem Wasserstoff.

Die Aufgabe wird zudem gelöst durch ein Verfahren zur Entfernung von Sauerstoff aus einem freien Sauerstoff enthaltenden Kohlenwasserstoffstrom durch katalytische Verbrennung, bei der der freie Sauerstoff enthaltende Kohlenwasserstoffstrom an einem wie vorstehend angegebenen Katalysator umgesetzt wird unter Erhalt eines sauerstoffabgereicherten Kohlenwasserstoffstromes.

Der erfindungsgemäß eingesetzte Katalysator hat dabei den Vorteil, dass er insbesondere die Umsetzung von Wasserstoff mit Sauerstoff katalysiert, ohne dass es dabei zu einer nennenswerten Umsetzung von Kohlenwasserstoff mit dem freien Sauerstoff kommt. Im Falle der Herstellung von Butadien aus Buten oder n-Butan kommt es dabei vorzugsweise nicht zu einer Umsetzung des Butadiens mit dem freien Sauerstoff.

Ein weiterer Vorteil des Einsatzes des erfindungsgemäßen Katalysators ist seine Stabilität gegenüber Wasser im Feed, insbesondere bei 5 bis 30 % Wasser im Feed.

Der erfindungsgemäße Katalysator enthält Zeolith A als Träger. Dabei liegen, bezogen auf den Träger, vorzugsweise mindestens 80 Gew.-%, besonders bevorzugt mindestens 90 Gew.-%, insbesondere mindestens 95 Gew.-% Zeolith A im Träger vor. Insbesondere ist der Träger ganz aus Zeolith A aufgebaut.

Zeolith A ist ein synthetisches, kristallines Alumosilikat und hat in seiner hydratisierten Natrium-Form die Summenformel Na₁₂((AlO₂)₁₂(SiO₂)₁₂) x 27 H₂O. Die Bezeichnung "Zeolith A" umfasst verschiedene Varianten dieser Verbindung, die alle das gleiche Alumosilikat-Gitter aufweisen. Sie können jedoch anstelle von Natriumionen andere Ionen wie Kalium oder Calcium enthalten. Auch wasserarme oder wasserfreie Formen werden erfindungsgemäß zu Zeolith A gezählt. Andere Bezeichnungen sind Molsieb A, Molekularsieb A, LTA (Linde-Typ A), MS 5 A (mit Ca), MS 4 A (mit Na), NF3 A (mit K), Sasil^{®}.

Zeolith A hat eine Gerüststruktur aus AlO₄- und SiO₄-Tetraedern. Sie bilden ein kovalentes Gitter mit Hohlräumen, die in der Regel Wasser enthalten. AlOₐ- und SiO₄-Tetraeder liegen dabei im Mengenverhältnis 1 : 1 vor. Dabei sind im Wechsel Aluminium- und Silizium-Atome untereinander über Sauerstoffatome verbunden. Insgesamt ergibt sich eine negative Ladung, die durch ionische Verbindungen mit Kationen wie Natriumionen ausgeglichen wird. Als räumliche Struktur weist Zeolith A einen so genannten Sodalith-Käfig auf.

Der erfindungsgemäße Katalysator enthält 0,01 bis 0,5 Gew.-%, vorzugsweise 0,05 bis 0,4 Gew.-%, insbesondere 0,1 bis 0,3 Gew.-% Platin, bezogen auf den Katalysator. Er kann zudem Zinn enthalten, wobei das Gewichtsverhältnis Sn : Pt 0 bis 10, vorzugsweise 0 bis 7, besonders bevorzugt 0 bis 3 beträgt. Bei der Mitverwendung von Zinn beträgt das Gewichtsverhältnis Sn : zPt vorzugsweise 0,5 bis 10, besonders bevorzugt 0,7 bis 4, insbesondere 0,9 bis 1,1. Speziell bevorzugt ist ein Gewichtsverhältnis Sn : Pt von 1 : 1.

Der erfindungsgemäße Katalysator kann neben Platin und Zinn auch weitere Metalle enthalten, beispielsweise Alkali- und/oder Erdalkalimetall-Verbindungen, vorzugsweise in Mengen < 2 Gew.-%, insbesondere < 0,5 Gew.-%, bezogen auf den Katalysator. Besonders bevorzugt enthält der Katalysator ausschließlich Platin und gegebenenfalls Zinn als Aktivmetalle.

Im fertigen Katalysator beträgt die BET-Oberfläche 20 bis 40 m²/g.

Der Katalysator kann dabei in beliebiger geeigneter Form eingesetzt werden. Vorzugsweise wird er als Formkörper mit einem mittleren Durchmesser im Bereich von 1 bis 10 mm, besonders bevorzugt 2 bis 8 mm, insbesondere 2,5 bis 5 mm eingesetzt. Der Formkörper kann dabei jede beliebige geeignete Form aufweisen, er kann als Strang, Tablette, Granulat, Split oder vorzugsweise in Kugelform mit dem angegebenen mittleren Durchmesser vorliegen. Weitere mögliche Formkörper sind Ringtabletten, Zylinder, Sternstränge oder zahnradförmige Stränge.

Alternativ kann der Katalysator als Monolith vorliegen, wobei der Monolith den Katalysator als Washcoat auf einer Trägerstruktur aufweisen kann. Diese Trägerstruktur kann die dreidimensionale Struktur des Monolithen vorgeben. Beispielsweise kann die Trägerstruktur aus Cordierit aufgebaut sein.

Der Anteil des Washcoats am gesamten Monolithen beträgt vorzugsweise 0,5 bis 5 g/inch³.

Der erfindungsgemäße Katalysator kann durch beliebige geeignete Verfahren hergestellt werden. Vorzugsweise wird er durch Tränken des Trägers mit einer Lösung einer Platinverbindung und gegebenenfalls einer Zinnverbindung und anschließendes Trocknen und Calcinieren hergestellt. Beispielsweise kann Platinnitrat als wässrige Lösung zum Tränken des Trägers eingesetzt werden. An die Tränkung kann sich eine Trocknung, vorzugsweise bei 80 bis 150°C und ein Calcinieren, vorzugsweise bei 200 bis 500°C, anschließen. Getrocknet wird vorzugsweise für einen Zeitraum im Bereich von 1 bis 100 Stunden, besonders bevorzugt 5 bis 20 Stunden. Das Calcinieren wird vorzugsweise für einen Zeitraum von 1 bis 20 Stunden, besonders bevorzugt 2 bis 10 Stunden, durchgeführt.

An die eigentliche Katalysatorherstellung kann sich eine Silylierung anschließen, beispielsweise durch Verwendung einer wässrigen kolloidalen Dispersion sehr kleiner Siliziumdioxid-Partikel, wie sie beispielsweise unter der Bezeichnung Ludos^{®} von der Helm AG verfügbar sind. Auch diese Silylierung kann durch Tränkung mit nachfolgender Trocknung und Calcinierung, wie sie vorstehend beschrieben ist, erfolgen.

Der erfindungsgemäße Katalysator zeichnet sich insbesondere durch eine Langzeitstabilität aus, speziell bei der Butan- oder Buten-Dehydrierung zur Herstellung von Butadien, wobei aus dem butadienhaltigen Produktstrom freier Sauerstoff abgetrennt werden soll.

Die katalytische Sauerstoffentfernung kann dabei grundsätzlich in allen aus dem Stand der Technik bekannten Reaktortypen und Fahrweisen durchgeführt werden, beispielsweise im Wirbelbett, Hordenofen, im Festbettrohr- oder Rohrbündelreaktor oder im Plattenwärmetauscherreaktor. Die erfindungsgemäße Entfernung von Sauerstoff kann in einem separaten Reaktionsschritt durchgeführt werden, sie kann jedoch auch mit einer oxidativen Dehydrierung in einem Reaktor kombiniert werden, wobei in einem Reaktor zwei Katalysatoren vorliegen.

Das erfindungsgemäße Verfahren kann zur Entfernung von Sauerstoff aus beliebigen Kohlenwasserstoffströmen eingesetzt werden. Vorzugsweise wird es zur Entfernung von Sauerstoff aus Kohlenwasserstoffströmen eingesetzt, die C₂₋₆-, vorzugsweise C₃- oder C₄-Kohlenwasserstoffe enthalten. Besonders bevorzugt wird das Verfahren auf Butadien oder Propen enthaltende Kohlenwasserstoffströme angewandt.

Speziell bevorzugt sind im Kohlenwasserstoffstrom mindestens 80 Vol.-%, besonders bevorzugt mindestens 90 Vol.-% der Kohlenwasserstoffe C₃- und/oder C₄-Kohlenwasserstoffe.

Besonders bevorzugt wird ein butadienhaltiger Kohlenwasserstoffstrom eingesetzt, der aus der Herstellung von Butadien aus n-Butan oder n-Butenen stammt. Dabei wird im Allgemeinen zunächst eine nicht-oxidative katalytische n-Butan-Dehydrierung durchgeführt, an die sich eine oxidative Dehydrierung anschließt. Geeignete Verfahren sind beispielsweise in WO 2006/075025, WO 2006/050969, WO 2006/061202 oder WO 2006/066848 beschrieben.

Beispielsweise erfolgt die Herstellung von Butadien aus n-Butan durch Einspeisung eines n-Butan enthaltenden Einsatzgasstroms in mindestens einer ersten Dehydrier-Zone und nicht-oxidative katalytische Dehydrierung des n-Butans, wobei ein Produktgasstrom enthaltend n-Butan, 1-Buten, 2-Buten, Butadien, Wasserstoff, leichtsiedende Nebenbestandteile, gegebenenfalls Kohlenstoffoxide und gegebenenfalls Wasserdampf erhalten wird. Dieser Produktgasstrom wird zusammen mit einem sauerstoffhaltigen Gas in mindestens eine zweite Dehydrier-Zone zur oxidativen Dehydrierung geführt, wobei ein Produktgasstrom erhalten wird, der n-Butan, 2-Butene, Butadiene, leichtsiedende Nebenbestandteile, Kohlenstoffoxide sowie Wasserdampf enthält.

Der die oxidative Dehydrierung verlassende Produktgasstrom enthält neben Butadien und nicht zuvor abgetrenntem n-Butan noch Wasserstoff, Kohlenstoffoxide und Wasserdampf. Als Nebenbestandteile kann er noch Sauerstoff, Inertgas wie Stickstoff, Methan, Ethan, Ethen, Propan und Propen sowie sauerstoffhaltige Kohlenwasserstoffe, so genannte Oxigenate, enthalten.

Im Allgemeinen weist der die oxidative Dehydrierung verlassende Produktgasstrom 2 bis 40 Vol.-% Butadien, 5 bis 80 Vol.-% n-Butan, 0 bis 15 Vol.-% 2-Butene, 0 bis 5 Vol.-% 1-Buten, 5 bis 70 Vol.-% Wasserdampf, 0 bis 10 Vol.-% leichtsiedende Kohlenwasserstoffe (Methan, Ethan, Ethen, Propan und Propen), 0,1 bis 15 Vol.-% Wasserstoff, 0 bis 70 Vol.-% Inertgas, 0 bis 10 Vol.-% Kohlenstoffoxide, 2 bis 10 Vol.-% Sauerstoff und 0 bis 10 Vol.-% Oxigenate auf, wobei die Gesamtmenge der Inhaltsstoffe 100 Vol.-% ergibt. Oxigenate können dabei beispielsweise Essigsäure, Methacrolein, Maleinsäureanhydrid, Maleinsäure, Phthalsäureanhydrid, Propionsäure, Acetaldehyd, Acrolein, Formaldehyd, Ameisensäure, Benzaldehyd, Benzoesäure und Butyraldehyd sein. Daneben können in Spuren Acetylen, Propin und 1,2-Butadien enthalten sein.

Vorzugsweise wird die erfindungsgemäße Sauerstoffentfernung unmittelbar nach der oxidativen Dehydrierung durchgeführt.

Bevorzugt enthält der eingesetzte Kohlenwasserstoffstrom 3 bis 8 Gew.-%, besonders bevorzugt 3 bis 6 Gew.-% freien Sauerstoff.

Der freien Sauerstoff enthaltende Kohlenwasserstoffstrom kann eine zur Umsetzung mit dem freien Sauerstoff ausreichende Menge an freiem Wasserstoff enthalten. Fehlende Mengen oder die Gesamtmenge an benötigtem freiem Wasserstoff kann/können auf dem Kohlenwasserstoffstrom zugesetzt werden. Bei dieser Reaktionsführung kann der freie Sauerstoff mit dem freien Wasserstoff umgesetzt werden, so dass kein nennenswerter Anteil des Kohlenwasserstoffs mit dem Sauerstoff umgesetzt wird.

In einer alternativen Ausführungsform enthält der freien Sauerstoff enthaltende Kohlenwasserstoffstrom keinen freien Wasserstoff und ihm wird auch kein freier Wasserstoff zugesetzt. In diesem Fall kann der freie Sauerstoff mit dem im freien Sauerstoff enthaltenden Kohlenwasserstoffstrom enthaltenden Kohlenwasserstoff oder mit zugesetztem Methanol, Erdgas und/oder Synthesegas als Reduktionsmittel umgesetzt werden.

Die Verfahrensführung kann dabei isotherm oder adiabatisch sein. Vorteil der Umsetzung des Wasserstoffs ist die Bildung von Wasser als Reaktionsprodukt. Das gebildete Wasser lässt sich durch Auskondensieren leicht abtrennen.

Zudem kann ein niedriger Reaktionsdruck von Vorteil sein, da hierdurch ein gesonderter Verdichtungsschritt nach der oxidativen Dehydrierung vermieden werden kann. Ein niedrigerer Umsetzungsdruck erlaubt eine weniger aufwendige Reaktorfertigung und ist aus Sicherheitsgründen vorteilhaft.

Das erfindungsgemäße Verfahren wird daher vorzugsweise bei einem Druck von 0,5 bis 3,0 bar (absolut), besonders bevorzugt 1,0 bis 2,0 bar (absolut) durchgeführt.

Die Umsetzung erfolgt vorzugsweise bei einer Temperatur im Bereich von 120 bis 500 °C, besonders bevorzugt 250 bis 400 °C.

Der Reaktortyp ist erfindungsgemäß nicht eingeschränkt. Beispielsweise kann die Umsetzung im Wirbelbett, im Hordenofen, im Festrohr- oder Rohrbündelreaktor oder im Plattenwärmetauscherreaktor erfolgen. Auch eine Kaskadierung von Wirbelschicht-Reaktoren ist denkbar.

Die bei der Reaktion entstehende Wärme kann über die Reaktorwände abgeführt werden. Zudem kann durch Strukturierung eines Festbettes des Katalysators mit Inert-Materialien die Bildung von hot spots vermindert werden.

Wird im erfindungsgemäßen Verfahren Wasserstoff im Unterschuss eingesetzt, so kann die Reaktion mit Wasserstoff dazu dienen, eine ausreichend hohe Temperatur für die notwendige Reaktion zwischen Kohlenwasserstoffen und Sauerstoff zu erreichen. Hierdurch kann eine Verkokung weitgehend vermieden werden.

Wird kein Wasserstoff, bzw. ein Wasserstoff-Unterschuss eingesetzt, so reagiert der Sauerstoff vorwiegend mit dem reaktivsten Molekül, beispielsweise Butadien. Hierdurch kommt es zu einer Bildung von Kohlenstoffoxiden und Wasser. Da die Umsetzung von Sauerstoff mit den Kohlenwasserstoffen bei niedriger Temperatur langsamer als mit Wasserstoff abläuft, wird zunächst der Wasserstoff vollständig verbraucht.

Die Erfindung wird durch die nachfolgenden Beispiele näher erläutert.

### Beispiele

### Beispiel 1

### Herstellung des Katalysators

Der Katalysator enthält 99,7 Gew.-% Zeolith-A, Molekularsieb 3A (der Roth-GmbH), 0,3 mm Typ 562 C, Perlform, Kugeln mit einem Durchmesser im Bereich von 2,5 bis 5 mm, sowie 0,3 Gew.-% Platin.

Zur Herstellung werden 1000 g Molekularsieb und 5,2 g Platinnitrat eingesetzt. Platinnitrat wird in Wasser gelöst, und die Lösung wird auf 460 ml Gesamtlösung aufgefüllt. Der Träger wird sodann zu 100 % seiner Wasseraufnahme getränkt. Hierzu wurde das Molekularsieb in zwei Porzellanschalen verteilt, die Tränklösung aufgetrennt, und es wurde gut gemischt.

Es schloss sich eine Trocknung für 16 Stunden bei 120°C im Umluft-Trockenschrank und eine Calcinierung für vier Stunden bei 400°C im Muffelofen an.

Zur Silylierung wurde der so erhaltene Katalysator in einem Becherglas vorgelegt und eine Lösung von Ludox und Wasser im Verhältnis 1 : 10 (Endkonzentration 4 Gew.-%) hergestellt. Die Menge wurde so bemessen, dass der Katalysator im Becherglas gut überdeckt werden konnte. In regelmäßigen Abständen wurde umgerührt und nach 40 Minuten über einen Faltenfilter abfiltriert. Es schloss sich wiederum eine Trocknung für 16 Stunden bei 120°C im Umluft-Trockenschrank und eine nachfolgende Calcinierung für 4 Stunden bei 400°C im Muffelofen an.

Die Elementaranalyse ergab einen Pt-Anteil im Katalysator von 0,27 Gew.-%.

### Beispiel 2

### Sauerstoffentfernung

Zum Einsatz kam ein Sauerstoff-Entfernungsreaktor einer Miniplant. Der Durchflussreaktor hatte eine Länge von 200 cm, einen äußeren Durchmesser von 25 cm, eine Wanddicke von 2 cm und einen Innendurchmesser von 21 cm. Er war aus Stahl aufgebaut.

Der Reaktor war mit drei externen Heizzonen ausgestattet, die für einen verbesserten Wärmetransfer von den Heizelementen zur Reaktorwand mit Kupferblöcken ausgestattet waren. Um ein adiabatisches System zu erhalten, wurden in der zweiten und dritten Heiz-Zone die Kupferblöcke entfernt und durch Isolationsmaterial ersetzt. In der ersten Aufheiz-Zone wurde eine Vorheiz-Zone eingesetzt, um die Einlassgastemperatur im Reaktor einzustellen. Die zweite und dritte Heiz-Zone wurden so eingestellt, dass Wärmeverluste weitestmöglich verhindert wurden. Der Röhrenreaktor wurde erst hinter dem Ende der ersten Heiz-Zone mit Katalysator befüllt. Ein pneumatisch betriebenes, multiples Thermoelement mit vier Mess-Stellen wurde zur Bestimmung der Temperaturprofile mit einer Auflösung von 2 cm im Katalysatorbett eingesetzt. Das Katalysatorbett wurde zwischen einem Inert-Material (Steatit) gepackt, das als Schutzbett diente. Sowohl isotherme als auch adiabatische Fahrweisen wurden untersucht.

Ein alternativer Reaktor im Labormaßstab wies eine Länge von 70 cm, einen Außendurchmesser von 25 cm, eine Wandstärke von 2 cm und einen inneren Durchmesser von 21 cm auf. Er war aus Stahl aufgebaut.

Typische Reaktionsbedingungen waren ein Katalysatorvolumen von 0,1 I, eine Katalysatormenge von 0,01 bis 0,1 kg, eine GHSV von 2000 bis 10000 Nl_{gas}l_{Kat}⁻¹h⁻¹, eine Einlass-Temperatur von 150 bis 410°C und ein Auslassdruck von 1,5 bis 2,5 bara.

Ein typischer Einlassgasstrom enthielt 15 bis 20 Vol.-% C₄-Kohlenwasserstoffe (70 Vol.-% Butadien und 30 Vol.-% Butan), 10 bis 20 Vol.-% Wasser, 5 bis 10 Vol.-% Wasserstoff, 50 bis 60 Vol.-% Stickstoff und 3 bis 5 Vol.-% Sauerstoff.

Bei einer Fahrweise ohne Wasserstoff wurde der Wasserstoff-Anteil durch Inertgas ersetzt.

Ziel des Verfahrens ist die Verringerung des Sauerstoffgehalts auf Werte von weniger als 100 ppm am Reaktorausgang. Für Verfahren ohne Wasserstoffzusatz beziehen sich die Ausbeuten auf CO₂ und Spuren von CO. Im Verfahren mit Wasserstoff-Einsatz beziehen sich die Ausbeuten auf CO₂ und CO, wie auf Dehydrierungsprodukte von Butadien (Buten-Isomere).

Bei einer Temperatur von 410°C, einem Druck von 0,5 bar/g und einer GHSV von etwa 3000 h⁻¹ wurden für den Katalysator aus Beispiel 1 ohne Zusatz von Wasserstoff Restsauerstoff-Gehalte von 97 ppm und bei Mitverwendung von Wasserstoff von 75 ppm bestimmt. Die Ausbeute betrug ohne Wasserstoff-Zusatz 4,0 %, mit Wasserstoff-Zusatz 3,6 %.

Wurde alternativ ein Katalysator eingesetzt, der 28 Gew.-% Kupfer auf Aluminiumoxid enthielt, so wurden 108 ppm Restsauerstoff ohne Wasserstoff-Zusatz und 100 ppm Restsauerstoff mit Wasserstoff-Zusatz bestimmt.

Der erfindungsgemäße Katalysator erfüllte die Anforderungen und zeigte gleichzeitig eine nur sehr geringe Bildung an Nebenprodukten.

### Beispiel 3

Es wurde die Miniplant aus Beispiel 2 eingesetzt und wie in Beispiel 2 vorgegangen, sofern nicht nachstehend anders angegeben.

| | | **Isotherm** | **Adiabat** |
|---|---|---|---|
| Gasmenge | | 377 Nl/h N_{2;} 150 Nl/h C₄.; 62 Nl/h H₂; 122 Nl/h Luft; 96 g/h H₂O | 377 Nl/h N₂; 150 Nl/h C₄.; 62 Nl/h H₂; 122 Nl/h Luft; 96 g/h H₂O |
| Rest 02 | ppm | 80 | 50 |
| C4-Verlust | % | 3,2 | 1,8 |
| Druck | barü | 0,5 | 0,5 |
| Stützheiz-Zone | °C | 200/200/200 | 375/530/530 |
| Material | cm | °C | °C |
| Inert | 0 | 190 | 305 |
| Inert | 2 | 194 | 305 |
| Inert | 4 | 203 | 306 |
| Inert | 6 | 222 | 307 |
| Inert | 8 | 242 | 309 |
| Inert | 10 | 260 | 311 |
| Katalysator | 12 | 277 | 312 |
| Katalysator | 14 | 327 | 330 |
| Katalysator | 16 | 405 | 384 |
| Katalysator | 18 | 466 | 443 |
| Katalysator | 20 | 477 | 482 |
| Katalysator | 22 | 479 | 503 |
| Katalysator | 24 | 464 | 519 |
| Katalysator | 26 | 449 | 527 |
| Katalysator | 28 | 429 | 533 |
| Katalysator | 30 | 411 | 535 |
| Katalysator | 32 | 399 | 535 |
| Katalysator | 34 | 390 | 534 |
| Katalysator | 36 | 379 | 533 |
| Katalysator | 38 | 365 | 532 |
| Inert | 40 | 352 | 531 |
| Inert | 42 | 335 | 530 |
| Inert | 44 | 323 | 524 |
| Inert | 46 | 321 | 523 |
| Inert | 48 | 318 | 522 |
| Inert | 50 | 317 | 520 |
| Inert | 52 | 313 | 519 |

## Patentansprüche

1. Katalysator, enthaltend auf Zeolith A als Träger 0,01 bis 0,5 Gew.-% Platin, bezogen auf den Katalysator, **dadurch gekennzeichnet, dass** die BET-Oberfläche des Katalysators 20 bis 40 m²/g beträgt.

2. Katalysator nach Anspruch 1, **dadurch gekennzeichnet, dass** der Platingehalt 0,05 bis 0,4 Gew.-% beträgt.

3. Katalysator nach Anspruch 1 oder 2, **dadurch gekennzeichnet, dass** er als Formkörper mit einem mittleren Durchmesser im Bereich von 1 bis 10 mm oder als Monolith vorliegt, wobei der Monolith den Katalysator als Washcoat auf einer Trägerstruktur aufweisen kann.

4. Verfahren zur Herstellung eines Katalysators nach einem der Ansprüche 1 bis 3 durch Tränken des Trägers mit einer Lösung einer Platinverbindung und anschließendes Trocknen und Calcinieren.

5. Verwendung eines Katalysators nach einem der Ansprüche 1 bis 4 zur Entfernung von Sauerstoff aus einem freien Sauerstoff enthaltenden Kohlenwasserstoffstrom durch katalytische Verbrennung in Anwesenheit oder Abwesenheit von freiem Wasserstoff.

6. Verfahren zur Entfernung von Sauerstoff aus einem freien Sauerstoff enthaltenden Kohlenwasserstoffstrom durch katalytische Verbrennung, bei der der freie Sauerstoff enthaltende Kohlenwasserstoffstrom an einem Katalysator nach einem der Ansprüche 1 bis 4 umgesetzt wird unter Erhalt eines sauerstoffabgereicherten Kohlenwasserstoffstromes.

7. Verfahren nach Anspruch 6, **dadurch gekennzeichnet, dass** der eingesetzte Kohlenwasserstoffstrom 3 bis 8 % freien Sauerstoff enthält.

8. Verfahren nach Anspruch 6 oder 7, **dadurch gekennzeichnet, dass** der freie Sauerstoff enthaltende Kohlenwasserstoffstrom eine zur Umsetzung mit dem freien Sauerstoff ausreichende Menge an freiem Wasserstoff enthält und/oder ihm diese zugesetzt wird und der freie Sauerstoff mit dem freiem Wasserstoff umgesetzt wird.

9. Verfahren nach Anspruch 6 oder 7, **dadurch gekennzeichnet, dass** der freien Sauerstoff enthaltende Kohlenwasserstoffstrom keinen freien Wasserstoff enthält und ihm kein freier Wasserstoff zugesetzt wird.

10. Verfahren nach Anspruch 9, **dadurch gekennzeichnet, dass** der freie Sauerstoff mit im freien Sauerstoff enthaltenden Kohlenwasserstoffstrom enthaltenem Kohlenwasserstoff oder mit zugesetztem Methanol, Erdgas und/oder Synthesegas als Reduktionsmittel umgesetzt wird.

11. Verfahren nach einem der Ansprüche 1 bis 10, **dadurch gekennzeichnet, dass** im Kohlenwasserstoffstrom mindestens 80 Vol.-% der Kohlenwasserstoffe C₃- und/oder C₄-Kohlenwasserstoffe sind.

## Claims

1. A catalyst comprising from 0.01 to 0.5% by weight of platinum, based on the catalyst, on zeolite A as support, wherein the BET surface area of the catalyst is from 20 to 40 m²/g.

2. The catalyst according to claim 1, wherein the platinum content is from 0.05 to 0.4% by weight.

3. The catalyst according to claim 1 or 2 which is present as shaped bodies having an average diameter in the range from 1 to 10 mm or as monolith, where the monolith can comprise the catalyst as washcoat on a support structure.

4. The process for producing a catalyst according to any of claims 1 to 3 by impregnation of the support with a solution of a platinum compound and subsequent drying and calcination.

5. The use of a catalyst according to any of claims 1 to 4 for removing oxygen from a hydrocarbon stream comprising free oxygen by catalytic combustion in the presence or absence of free hydrogen.

6. A process for removing oxygen from a hydrocarbon stream comprising free oxygen by catalytic combustion, wherein the hydrocarbon stream comprising free oxygen is reacted over a catalyst according to any of claims 1 to 4 to give an oxygen-depleted hydrocarbon stream.

7. The process according to claim 6, wherein the hydrocarbon stream used comprises from 3 to 8% of free oxygen.

8. The process according to claim 6 or 7, wherein the hydrocarbon stream comprising free oxygen comprises an amount of free hydrogen sufficient for reaction with the free oxygen and/or hydrogen is added thereto and the free oxygen is reacted with the free hydrogen.

9. The process according to claim 6 or 7, wherein the hydrocarbon stream comprising free oxygen does not comprise any free hydrogen and no free hydrogen is added thereto.

10. The process according to claim 9, wherein the free oxygen is reacted with hydrocarbon comprised in the hydrocarbon stream comprising free oxygen or with added methanol, natural gas and/or synthesis gas as reducing agent.

11. The process according to any of claims 1 to 10, wherein at least 80% by volume of the hydrocarbons in the hydrocarbon stream are C₃- and/or C₄-hydrocarbons.

## Revendications

1. Catalyseur, contenant sur une zéolithe A en tant que support 0,01 à 0,5 % en poids de platine, par rapport au catalyseur, **caractérisé en ce que** la surface BET du catalyseur est de 20 à 40 m²/g.

2. Catalyseur selon la revendication 1, **caractérisé en ce que** la teneur en platine est de 0,05 à 0,4 % en poids.

3. Catalyseur selon la revendication 1 ou 2, **caractérisé en ce qu'**il se présente sous la forme d'un corps moulé d'un diamètre moyen dans la plage allant de 1 à 10 mm ou sous la forme d'un monolithe, le monolithe pouvant comprendre le catalyseur sous la forme d'une couche d'imprégnateur sur une structure support.

4. Procédé de fabrication d'un catalyseur selon l'une quelconque des revendications 1 à 3 par imprégnation du support avec une solution d'un composé de platine, puis séchage et calcination.

5. Utilisation d'un catalyseur selon l'une quelconque des revendications 1 à 4 pour l'élimination d'oxygène d'un courant d'hydrocarbures contenant de l'oxygène libre par combustion catalytique en présence ou en absence d'hydrogène libre.

6. Procédé d'élimination d'oxygène d'un courant d'hydrocarbures contenant de l'oxygène libre par combustion catalytique, selon lequel le courant d'hydrocarbures contenant de l'oxygène libre est mis en réaction sur un catalyseur selon l'une quelconque des revendications 1 à 4 pour obtenir un courant d'hydrocarbures appauvri en oxygène.

7. Procédé selon la revendication 6, **caractérisé en ce que** le courant d'hydrocarbures utilisé contient 3 à 8 % d'oxygène libre.

8. Procédé selon la revendication 6 ou 7, **caractérisé en ce que** le courant d'hydrocarbures contenant de l'oxygène libre contient une quantité suffisante d'hydrogène libre pour la réaction avec l'oxygène libre et/ou celle-ci lui est ajoutée et l'oxygène libre est mis en réaction avec l'hydrogène libre.

9. Procédé selon la revendication 6 ou 7, **caractérisé en ce que** le courant d'hydrocarbures contenant de l'oxygène libre ne contient pas d'hydrogène libre et aucun hydrogène libre ne lui est ajouté.

10. Procédé selon la revendication 9, **caractérisé en ce que** l'oxygène libre est mis en réaction avec l'hydrocarbure contenu dans le courant d'hydrocarbures contenant de l'oxygène libre ou avec du méthanol, du gaz naturel et/ou un gaz de synthèse ajouté en tant que réducteur.

11. Procédé selon l'une quelconque des revendications 1 à 10, **caractérisé en ce qu'**au moins 80 % en volume des hydrocarbures dans le courant d'hydrocarbures sont des hydrocarbures en C₃ et/ou C₄.
